# EUROPEAN PATENT APPLICATION

(11) **EP 3 012 277 A1**
(43) Date of publication of application: **27.04.2016**
(21) Application number: 14812935.6
(22) Date of filing: 13.06.2014
(51) Int. Cl.: C08G 18/38, G02B 1/04, C07C 321/14, C07C 323/12

(54) **COMPOSITION FOR OPTICAL MATERIAL**

(30) Priority: 18.06.2013 JP 2013127617
(71) Applicant: Mitsubishi Gas Chemical Company, Inc., Tokyo 100-8324 (JP)
(72) Inventor: NISHIMORI, Yoshihiko, Tokyo 125-8601 (JP); KAMURA, Teruo, Tokyo 125-8601 (JP); HORIKOSHI, Hiroshi, Tokyo 125-8601 (JP)
(74) Representative: Müller-Boré & Partner Patentanwälte PartG mbB
(86) International application number: PCT/JP2014/065710
(87) International publication number: WO 2014/203812

(57) **Abstract**

According to the present invention, a composition for an optical material contains a compound (a), a compound (b), and a polyisocyanate compound (c), and can suppress the generation of striae defects. The compound (a) is a compound having a structure represented by formula (1). 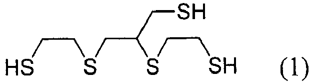 The compound (b) is a compound having a structure represented by formula (2).

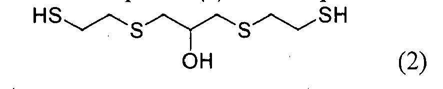

## Description

### TECHNICAL FIELD

The present invention relates to a composition for optical materials comprising a specific polythiol compound and a polyisocyanate compound, and further relates to an optical material made of a thiourethane resin obtained by polymerizing the composition for optical materials.

### BACKGROUND ART

Plastic materials are lightweight, highly tough and easy to be dyed, and therefore are widely used recently for various types of optical materials, particularly eyeglass lenses. Optical materials, particularly eyeglass lenses, are specifically required to have, as main physical properties, low specific gravity, high transparency and low yellowness, and as optical properties, high refractive index and high Abbe number. With respect to thiourethane resins for optical lenses and plastic lenses produced by utilizing the same, many patent applications were filed, and uncolored lenses having a high refractive index and high transparency have been proposed.

However, in the case of powerful lenses made of thiourethane resins, striae may be generated, and reduction in the yield is caused thereby. For this reason, proposes about the type of a polymerization catalyst and a method for addition thereof, and about a stabilizer for an isocyanate which is chemically unstable have been made (Patent Documents 1-3). However, the improvement of striae of thiourethane resins is not necessarily sufficient, and it is desired to further improve the yield.

### PRIOR ART DOCUMENTS

### PATENT DOCUMENTS

Patent Document 1: Japanese Laid-Open Patent Publication No. H10-81726
Patent Document 2: Japanese Laid-Open Patent Publication No. 2002-275232
Patent Document 3: Japanese Laid-Open Patent Publication No. H07-104101

### SUMMARY OF THE INVENTION

### PROBLEMS TO BE SOLVED BY THE INVENTION

The problem to be solved by the present invention is to provide a composition for optical materials comprising a polythiol compound and a polyisocyanate compound, which can improve reduction in yield due to striae defects, and an optical material obtained by polymerizing and curing the composition.

### MEANS FOR SOLVING THE PROBLEMS

The present inventor diligently made researches in order to solve the above-described problems and determined that striae are caused by convection at an early stage of cast molding with a low viscosity, and found that use of a polythiol compound comprising a compound with a hydroxy group which has excellent reactivity with an isocyanate compared to reactivity with a thiol group can increase the rate of viscosity rise at the early stage of cast molding and suppress striae defects caused by convection.

Specifically, the above-described problems can be solved by the present invention as described below:
<1> A polythiol comprising: a compound (a) as the main component; and a compound (b) in an amount of 0.5 to 10% by mass, wherein the compound (a) is a compound having a structure represented by formula (1) below: 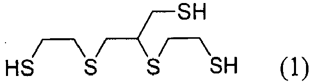 and wherein the compound (b) is a compound having a structure represented by formula (2) below: 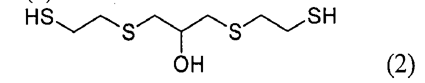
<2> A composition for optical materials, which contains a polythiol comprising: a compound (a) as the main component; and a compound (b) in an amount of 0.5 to 10% by mass, and a polyisocyanate compound (c), wherein the compound (a) is a compound having a structure represented by formula (1) below: 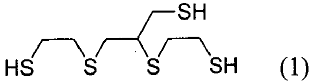 and wherein the compound (b) is a compound having a structure represented by formula (2) below: 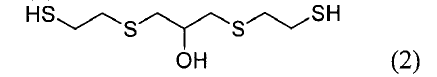
<3> The composition for optical materials according to item <2>, wherein the molar ratio of NCO groups to the total amount of SH groups and OH groups (NCO/(SH+OH)) is 0.7 to 1.5.
<4> An optical material obtained by polymerizing and curing the composition for optical materials according to item <2> or <3>.
<5> A lens comprising the optical material according to item <4>.

### ADVANTAGEOUS EFFECT OF THE INVENTION

According to the present invention, it is possible to produce an optical material made of a thiourethane resin which is free of optical distortion and has excellent property balance at a high yield rate, and it is possible to improve the productivity of optical materials.

### EMBODIMENTS FOR CARRYING OUT THE INVENTION

In the present invention, a polythiol, which comprises: the aforementioned compound (a) as the main component; and the aforementioned compound (b) in an amount of 0.5 to 10% by mass, is used. The ratio of the compound (b) in the polythiol is preferably 0.5 to 7% by mass, more preferably 0.5 to 5% by mass, and particularly preferably 0.8 to 5% by mass. When the ratio of the compound (b) in the polythiol is too low, the effect of improving striae is not exerted, and when the ratio is too high, resin properties including refractive index and heat resistance may be deteriorated.

The ratio of the compound (a) in the polythiol is 70 to 90% by mass, preferably 73 to 86% by mass, and more preferably 76 to 83% by mass.

The compound (c) to be used in the present invention is not particularly limited as long as it is a compound having at least two isocyanates in one molecule. Specific examples thereof include diethylene diisocyanate, tetramethylene diisocyanate, hexamethylene diisocyanate, trimethylhexamethylene diisocyanate, cyclohexane diisocyanate, 1,3-bis(isocyanatemethyl)cyclohexane, 1,4-bis(isocyanatemethyl)cyclohexane, isophorone diisocyanate, 2,6-bis(isocyanatemethyl)decahydronaphthalene, lysine triisocyanate, tolylene diisocyanate, o-tolidine diisocyanate, diphenylmethane diisocyanate, diphenylether diisocyanate, 3-(2'-isocyanatecyclohexyl)propylisocyanate, isopropylidene bis(cyclohexyl isocyanate), 2,2'-bis(4-isocyanatephenyl)propane, triphenylmethane triisocyanate, bis(diisocyanatetolyl)phenylmethane, 4,4',4"-triisocyanate-2,5-dimethoxyphenylamine, 3,3'-dimethoxybenzidine-4,4'-diisocyanate, 1,3-phenylene diisocyanate, 1,4-phenylene diisocyanate, 4,4'-diisocyanatebiphenyl, 4,4'-diisocyanate-3,3'-dimethylbiphenyl, dicyclohexylmethane-4,4'-diisocyanate, 1,1 '-methylenebis(4-isocyanatebenzene), 1,1'-methylenebis(3-methyl-4-isocyanatebenzene), m-xylylene diisocyanate, p-xylylene diisocyanate, m-tetramethyl xylylene diisocyanate, p-tetramethyl xylylene diisocyanate, 1,3-bis(2-isocyanate-2-propyl)benzene, 2,6-bis(isocyanatemethyl)naphthalene, 1,5-naphthalene diisocyanate, bis(isocyanatemethyl)tetrahydrodicyclopentadiene, bis(isocyanatemethyl)dicyclopentadiene, bis(isocyanatemethyl)tetrahydrothiophene, bis(isocyanatemethyl)norbornene, bis(isocyanatemethyl)adamantane, thiodiethyl diisocyanate, thiodipropyl diisocyanate, thiodihexyl diisocyanate, bis [(4-isocyanatemethyl)phenyl] sulfide, 2,5-diisocyanate-1,4-dithiane, 2,5-diisocyanatemethyl-1,4-dithiane, 2,5-diisocyanatemethylthiophene, dithiodiethyl diisocyanate and dithiodipropyl diisocyanate.

Among them, at least one compound selected from isophorone diisocyanate, tolylene diisocyanate, diphenylmethane diisocyanate, hexamethylene diisocyanate, m-xylylene diisocyanate, p-xylylene diisocyanate, m-tetramethyl xylylene diisocyanate, p-tetramethyl xylylene diisocyanate, 1,3-bis(isocyanatemethyl)cyclohexane, 1,4-bis(isocyanatemethyl)cyclohexane, bis(isocyanatemethyl)norbornene and 2,5-diisocyanatemethyl-1,4-dithiane is a preferred specific example. Isophorone diisocyanate, tolylene diisocyanate, diphenylmethane diisocyanate, hexamethylene diisocyanate, 1,3-bis(isocyanatemethyl)cyclohexane and m-xylylene diisocyanate are more preferred, isophorone diisocyanate, m-xylylene diisocyanate and 1,3-bis(isocyanatemethyl)cyclohexane are even more preferred, and m-xylylene diisocyanate is the most preferred compound.

However, the compound (c) to be used in the present invention is not limited to the above-described examples, and these compounds may be used solely, or two or more of them may be used in combination.

The ratio (molar ratio) of the compound (c) to be used in the present invention is not particularly limited, but usually, the molar ratio of NCO groups to the total amount of SH groups and OH groups (NCO/(SH+OH)) is 0.7 to 1.5, preferably 0.7 to 1.3, and more preferably 0.8 to 1.2.

At the time of obtaining an optical material by polymerizing and curing the composition for optical materials of the present invention, with the polythiol comprising the compound (a) as the main component (mixture in which a structural isomer, an oligomer and a low-molecular weight are mixed), the polythiol comprising the compound (b) as the main component (mixture in which a structural isomer, an oligomer and a low-molecular weight are mixed) and the compound (c), a polymerization catalyst, and according to need, additives and the like are preferably mixed. The ratio of the total of the polythiol comprising the compound (a) as the main component, the polythiol comprising the compound (b) as the main component and the compound (c) in the composition for optical materials of the present invention is usually at least 80% by mass, preferably at least 90% by mass, and more preferably at least 95% by mass.

Moreover, depending on purposes, various substances such as a publicly-known chain extender, crosslinking agent, photostabilizer, ultraviolet absorber, antioxidant, oil-soluble dye, filler and internal mold release agent may be added. In order to adjust the reaction rate, a publicly-known reaction catalyst used in the production of polythiourethane may be suitably added. When producing a plastic lens from the composition for optical materials of the present invention, it is usually carried out by cast polymerization.

In the method for producing the composition for optical materials of the present invention, after mixing components, the defoaming treatment must be carried out. It is preferred to carry out the defoaming treatment under suitable conditions prior to the polymerization and curing of the composition for optical materials in terms of preventing defects such as deterioration of transparency of the optical material due to influence of low-boiling components such as carbon dioxide generated by a reaction between water and isocyanate groups contained in the composition. The degree of vacuum is 0.01 to 50 Torr, preferably 0.01 to 30 Torr, more preferably 0.1 to 10 Torr, and even more preferably 0.1 to 5 Torr.

A cast molding solution obtained in this way can be purified by filtering impurities, etc. with a filter or the like immediately prior to polymerization and curing. It is desirable to purify the composition for optical materials by filtering impurities, etc. with a filter in terms of further improving the quality of the optical material of the present invention. The pore diameter of the filter to be used herein is about 0.05 to 10 µm, and a filter having a pore diameter of 0.1 to 1.0 µm is generally used. Regarding the material of the filter, PTFE, PET, PP, etc. are suitably used.

The temperature and time for polymerization vary depending on the type of a monomer and additives, but the temperature is -10°C to 160°C, and usually -10°C to 140°C. The polymerization can be conducted by carrying out a step of holding the composition at a predetermined polymerization temperature for a predetermined amount of time, a step of increasing the temperature at a rate of 0.1 °C to 100°C/h and a step of decreasing the temperature at a rate of 0.1 °C to 100°C/h, or a combination of these steps.

Further, it is preferred to anneal the material at a temperature of 50°C to 150°C for about 5 minutes to 5 hours after the polymerization is completed in terms of eliminating distortion of the optical material. Moreover, it can be subjected to a surface treatment such as dyeing, hard coating, antireflection treatment and imparting antifog properties, antifouling properties, impact resistance, etc. according to need. The optical material of the present invention has heat resistance of preferably 82°C or higher, and more preferably 85°C or higher. Further, the rate of striae generation of the optical material is preferably 15% or lower, and more preferably 10% or lower.

### EXAMPLES

Hereinafter, the present invention will be specifically described by way of synthesis examples, working examples and comparative examples, but the present invention is not limited only to these working examples. Note that cast molding solutions (compositions for optical materials) and resins (optical materials) obtained were evaluated according to the below-described methods.
- Quantitation of compound (a) and compound (b) in polythiol
   Quantitation was carried out according to the absolute calibration curve method using a capillary gas chromatograph (manufactured by Shimadzu Corporation, GC2010, detector: flame ionization detector (FID)) equipped with DB-5MS (Agilent Technologies).
- Viscosity of cast molding solution
   The viscosity at 20°C was measured using a Brookfield viscometer (manufactured by Toki Sangyo Co., Ltd., type TV10M).
- Rate of striae generation of optical material made of thiourethane resin
   Light from a mercury lamp as a light source was transmitted through 100 lenses made of thiourethane resin (diameter: 75 mm, -15D) prepared, the transmitted light was projected onto a white board, and the rate of striae generation was evaluated based on the presence or absence of stripe-like contrast.
- Refractive index of optical material made of thiourethane resin
   Regarding the refractive index of optical materials, the refractive index at the e-line at 25°C was measured using a digital precision refractometer (Shimadzu Corporation, KPR-200).
- Heat resistance (Tg) of optical material made of thiourethane resin
   A sample was cut to have a thickness of 3 mm, and the TMA measurement (Seiko Instruments Inc., TMA/SS6100) was carried out by adding 10 g of weight to a pin (ϕ: 0.5 mm) and elevating the temperature from 30°C at a rate of 2.5°C/min to measure the softening point.

### Synthesis Example 1

76.0 parts by mass of water and 90.0 parts by mass (1.08 mol) of aqueous solution of sodium hydroxide (48% by mass) were put into a 2L four-neck reaction flask equipped with a stirring machine, a reflux cooling tube, a nitrogen gas purge tube and a thermometer. 169 parts by mass (2.16 mol) of 2-mercaptoethanol was added dropwise thereto at 30°C over 30 minutes, and after that, 99.9 parts by mass (1.08 mol) of epichlorohydrin was added dropwise thereto at the same temperature over 3 hours, and the mixture was matured for 1 hour. Next, 450.1 parts by mass (4.32 mol) of water containing hydrochloric acid (36% by mass) and 304.5 parts by mass (4.00 mol) of thiourea were added thereto, and the mixture was refluxed at 110°C for 8 hours to provide a thiouronium salt. After it was cooled to 50°C, 450.0 parts by mass of toluene and 298 parts by mass (5.21 mol) of aqueous solution of ammonia (28% by mass) were added thereto to perform hydrolysis, thereby obtaining a toluene solution of polythiol containing the compound (a) as the main component. The toluene solution was washed with an acid and then with water, and toluene and a slight amount of water were removed under reduced pressure with heating. After that, it was filtered, thereby obtaining 270.2 parts by mass of a polythiol containing the compound (a) as the main component (including a mixture in which a structural isomer (about 10%), an oligomer (several percent) and a low-molecular weight component are mixed).

### Synthesis Example 2

76.0 parts by mass of water and 90.0 parts by mass (1.08 mol) of aqueous solution of sodium hydroxide (48% by mass) were put into a 2L four-neck reaction flask equipped with a stirring machine, a reflux cooling tube, a nitrogen gas purge tube and a thermometer. 169 parts by mass (2.16 mol) of 2-mercaptoethanol was added dropwise thereto at 5°C over 10 minutes, and after that, 99.9 parts by mass (1.08 mol) of epichlorohydrin was added dropwise thereto at the same temperature over 1 hour, and the temperature was increased to 30°C. Next, 450.1 parts by mass (4.32 mol) of water containing hydrochloric acid (36% by mass) and 206 parts by mass (2.70 mol) of thiourea were added thereto, and the mixture was refluxed at 110°C for 30 minutes to provide a thiouronium salt. After it was cooled to 30°C, 450.0 parts by mass of toluene and 300 parts by mass (5.25 mol) of aqueous solution of ammonia (28% by mass) were added thereto to perform hydrolysis at 50°C, thereby obtaining a toluene solution of polythiol containing the compound (b) as the main component. The toluene solution was washed with an acid and then with water, and toluene and a slight amount of water were removed under reduced pressure with heating. After that, it was filtered, thereby obtaining 259.5 parts by mass of a polythiol containing the compound (b) as the main component (including a mixture in which the compound (a) (about 20%), an oligomer (several percent) and a low-molecular weight component are mixed).

### Comparison of reaction rate

48 parts by mass (0.184 mol) of the polythiol containing the compound (a) as the main component synthesized in Synthesis Example 1, 52 parts by mass (0.276 mol) of m-xylylene diisocyanate and 0.015 parts by mass of di-n-butyltin dichloride were mixed together at 20°C to be dissolved. The viscosity at that time was 10 mPa·s. The mixture was further retained at 20°C, and 2 hours later, the viscosity was 19 mPa·s. The difference in the viscosity 2 hours after mixing was 9 mPa·s.

46.4 parts by mass (0.190 mol) of the polythiol containing the compound (b) as the main component synthesized in Synthesis Example 2, 53.6 parts by mass (0.285 mol) of m-xylylene diisocyanate and 0.015 parts by mass of di-n-butyltin dichloride were mixed together at 20°C to be dissolved. The viscosity at that time was 10 mPa·s. The mixture was further retained at 20°C, and 2 hours later, the viscosity was 29 mPa·s. The difference in the viscosity 2 hours after mixing was 19 mPa·s.

Since the difference in the viscosity change 2 hours later between the compound (a) and the compound (b) was 10 mPa·s, it was confirmed that the compound (a) and the compound (b) do not have the relationship of 2 types of polythiol compounds with different rates of reaction with a polyisocyanate compound defined in Japanese Laid-Open Patent Publication No. H07-104101.

In the below-described Examples and Comparative Examples, the polythiol containing the compound (a) as the main component synthesized in Synthesis Example 1 was mixed with the polythiol containing the compound (b) as the main component synthesized in Synthesis Example 2 to prepare polythiols containing the compound (b) at respective concentrations.

### Example 1

52 parts by mass of m-xylylene diisocyanate, 0.015 parts by mass of di-n-butyltin dichloride, 0.1 parts by mass of Zelec UN (manufactured by Stepan) and 0.05 parts by mass of Biosorb 583 (manufactured by Kyodo Chemical Co., Ltd.) were mixed together to be dissolved. 48 parts by mass of a polythiol containing 75% by mass of the compound (a) and 6.9% by mass of the compound (b) was added thereto to be mixed at 15°C, thereby producing a mixed homogeneous solution. This mixed homogeneous solution was subjected to deforming at 600Pa for 30 minutes. After that, it was filtered using a 1-µm PTFE filter and then injected into a mold for -15D made of various glass molds and a tape. This mold was put into an oven, and the temperature was gradually increased from 10°C to 120°C to perform polymerization for 20 hours. After the polymerization was completed, the mold was taken out from the oven, and a resin was obtained by being released from the mold. The obtained resin was further subjected to annealing at 130°C for 2 hours, thereby obtaining an optical material (lens) made of thiourethane resin. The molar ratio of NCO groups to the total amount of SH groups and OH groups of the raw material (NCO/(SH+OH)), the viscosity of the cast molding solution after preserved at 15°C for 7 hours, and evaluation results of the optical material made of thiourethane resin were as shown in Table 1.

### Examples 2-4, Comparative Examples 1-4

Optical materials made of thiourethane resin were prepared in a manner similar to that in Example 1, except that polythiols containing the compound (b) at respective concentrations were used instead of the polythiol used in Example 1. The molar ratio of NCO groups to the total amount of SH groups and OH groups of the raw material (NCO/(SH+OH)), the viscosity of the cast molding solution 7 hours later, and evaluation results of the optical material made of thiourethane resin were as shown in Table 1.

**Table 1**

| | Concentration of compound (a) in polythiol (%) | Concentration of compound (b) in polythiol (%) | NCO/(SH+OH) | Viscosity after 7 hours | Rate of striae generation (%) | Refractive index of resin | Heat resistance (°C) |
|---|---|---|---|---|---|---|---|
| Example 1 | 75 | 6.9 | 1.0 | 153 | 3 | 1.664 | 85 |
| Example 2 | 78 | 2.8 | 1.0 | 141 | 2 | 1.665 | 86 |
| Example 3 | 81 | 1.0 | 1.0 | 126 | 4 | 1.665 | 88 |
| Example 4 | 82 | 0.6 | 1.0 | 117 | 7 | 1.665 | 87 |
| Comparative Example 1 | 83 | 0.4 | 1.0 | 106 | 16 | 1.665 | 87 |
| Comparative Example 2 | 85 | 0.2 | 1.0 | 104 | 17 | 1.665 | 88 |
| Comparative Example 3 | 68 | 13 | 1.0 | 165 | 4 | 1.663 | 80 |
| Comparative Example 4 | 60 | 23 | 1.0 | 191 | 5 | 1.661 | 81 |

According to the results of the Examples and Comparative Examples, it was confirmed that the rate of viscosity rise was increased and the viscosity after 7 hours was increased with the increase of the compound (b) in the composition for optical materials, and with this, the rate of striae generation was reduced. In particular, when the amount of the compound (b) in the polythiol was more than 0.5% by mass, the rate of striae generation was significantly reduced. Accordingly, it was found that striae defects can be reduced by using a composition for optical materials in which a polythiol containing at least 0.5% by mass of the compound (b) is used. Meanwhile, the refractive index of resin and the heat resistance were reduced with the increase of the compound (b), and when the amount of the compound (b) was more than 10% by mass, significant reduction in physical properties was confirmed. Specifically, it was found that it is possible to provide an optical material made of a polyurethane resin having excellent property balance, in which striae defects are suppressed, by using a composition for optical materials in which a polythiol containing 0.5 to 10% by mass of the compound (b) is used.

## Claims

1. A polythiol comprising: a compound (a) as the main component; and a compound (b) in an amount of 0.5 to 10% by mass, wherein the compound (a) is a compound having a structure represented by formula (1) below: 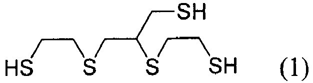 and wherein the compound (b) is a compound having a structure represented by formula (2) below:

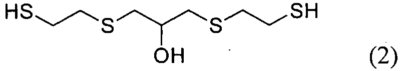

2. A composition for optical materials, which contains a polythiol comprising: a compound (a) as the main component; and a compound (b) in an amount of 0.5 to 10% by mass, and a polyisocyanate compound (c), wherein the compound (a) is a compound having a structure represented by formula (1) below: 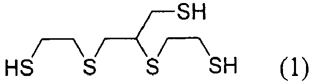 and wherein the compound (b) is a compound having a structure represented by formula (2) below:

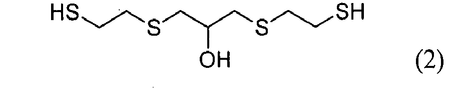

3. The composition for optical materials according to claim 2, wherein the molar ratio of NCO groups to the total amount of SH groups and OH groups (NCO/(SH+OH)) is 0.7 to 1.5.

4. An optical material obtained by polymerizing and curing the composition for optical materials according to claim 2 or 3.

5. A lens comprising the optical material according to claim 4.
